# EUROPEAN PATENT SPECIFICATION

(11) **EP 1 948 781 B1**
(45) Date of publication and mention of the grant of the patent: **30.12.2009**
(21) Application number: 06799344.4
(22) Date of filing: 19.10.2006
(51) Int. Cl.: C12N 1/00, C12N 1/16, C12N 1/20

(54) **MICROORGANISMS HAVING BAD SMELL REMOVAL ACTIVITY OF ORGANIC WASTE AND USE THEREOF**
MIKROORGANISMEN MIT AKTIVITÄT ZUR BESEITIGUNG VON SCHLECHTEM GERUCH ORGANISCHER ABFÄLLE UND VERWENDUNG DAVON
MICRO-ORGANISMES AYANT UNE ACTIVITE D'ELIMINATION DE LA MAUVAISE ODEUR DES DECHETS ORGANIQUES ET UTILISATION DE CEUX-CI

(30) Priority: 22.10.2005 KR 20050099940; 01.11.2005 KR 20050103923; 26.09.2006 KR 20060093709; 26.09.2006 KR 20060093724; 26.09.2006 KR 20060093713; 28.09.2006 KR 20060094706; 28.09.2006 KR 20060094687; 10.10.2006 KR 20060098303
(43) Date of publication of application: 30.07.2008
(73) Proprietor: Park, Se Joon, Seocho-gu, Seoul 137-921 (KR)
(72) Inventor: Park, Se Joon, Seocho-gu, Seoul 137-921 (KR)
(74) Representative: Schnabel, Jörg
(86) International application number: PCT/KR2006/004270
(87) International publication number: WO 2007/046650

(56) References cited:
- EP-A- 1 374 695
- EP-A- 1 374 697
- KR-A- 970 043 015
- KR-A- 19980 056 781
- KR-A- 20020 027 818
- US-A- 6 051 219
- US-B1- 6 649 397

## Description

### TECHNICAL FIELD

The present invention relates to a novel microorganism having the effect of removing an offensive odor from organic waste and the use thereof, and more particularly to a microorganism having the functions of preventing or removing the offensive odor of organic waste, killing insects and fungi, preventing decomposition, and promoting digestion and fermentation; and the use thereof.

### BACKGROUND ART

Sewage wastewater and waste, which are inevitable consequence of human life, and organic waste such as livestock manure generated from animals or livestock, are biochemically degraded by the action of minerals, metals, salts and microorganisms in the soil under suitable humidity and temperature, thus causing various odors. The generated odors are hydrogen sulfide, carbolic acids or compounds thereof, and other irritating gaseous substances, which give unpleasant sensations in daily life, and among them, inorganic substances and alkaline substances are substantially odorless, but almost all the organic substances generate odors. Particularly, sulfide compounds and nitrogen compounds are the main causes of odors.

Prior methods of removing the odors thus generated include a masking method, an adsorption method, a neutralization method, sterilization method and the like. The masking method is a method of generating a distinctive smell stronger than an offensive odor, such that the offensive odor is not felt, but this method requires expensive fragrances and it is difficult to fundamentally remove the offensive odor. The adsorption method is a physical method of adsorbing an offensive odor onto activated carbon or the like while discharging the offensive odor to the outdoors by an exhaust system, and has shortcomings in that high construction cost is required and high maintenance cost is incurred because expensive activated carbon must be periodically used. Also, the neutralization method is a chemical method of neutralizing an offensive odor into an acidic or alkaline substance, and enables an offensive odor to temporarily disappear during the use thereof. However, the neutralization method has shortcoming in that the disappearance of the offensive odor does not last long, it is difficult to treat an odor-causing substance having both acidic and basic groups, and it cannot have any effect if an odor-causing substance is neutral. The sterilization method is a method of killing bacteria themselves degrading organic substances, so as to prevent the decomposition of the organic substances and the generation of odors from the organic substances, and has a shortcoming in that it requires an expensive bactericide or preservative for maintaining an odorless state for a long time. Particularly, because the sterilization method aims to prevent the decomposition or fermentation of organic substances from occurring, it cannot be used in cases where a desired substance is obtained only by odors that are caused by the decomposition or fermentation of organic waste.

Thus, it will be preferable in economic terms to use a biological method of oxidizing and decomposing organic waster using microorganisms such as bacteria.

In Korean Patent Registration Nos. 10-0536456 and 10-0581738, a novel yeast strain and the genus *Bacillus* strain that ferment organic waste were isolated and identified and these strains were confirmed to have the effects of preventing offensive odors and killing and inhibiting harmful insects and pathogenic bacteria.

Also, most fermented foods, including alcoholic drinks, breads, vinegars, fermented soybean foods (soy sauce, soybean paste, thick soypaste mixed with red peppers, etc.), fermented milk products (cheese, butter, yogurt, etc.), salted foods (Kimchi, salted fish, etc.), red ginseng, and skates, are made by a number of microorganisms produced in nature and have characteristic odors. Recently, as interest in fermentation has increased, various studies, including the preparation of fermented ginseng, Chungkookjang confectionary and lactic acid fermented foods, the characteristic odors of which were removed, have been conducted.

Accordingly, the present inventors have made extensive efforts to develop a method for removing an offensive odor from organic waste using microorganism. As a result, the present inventors have isolated and identified a novel microorganism having the efficiency in removing the offensive odor of organic waste and found that the novel microorganism shows the effects of removing the offensive odor of organic waste, killing insects and fungi, preventing decomposition, and promoting fermentation, thereby completing the present invention.

### SUMMARY OF INVENTION

It is an object of the present invention to provide a microorganism having the efficiency in removing an offensive odor from organic waste.

Another object of the present invention is to provide a microbial agent for the fermentation of organic waste, which contains said microorganism.

Still another object of the present invention is to provide an agent for preventing or removing an offensive odor from organic waste, which contains said microorganism.

Yet another object of the present invention is to provide an insecticide, a microbicide and a preservative, which contain said microorganism.

Yet still another object of the present invention is to provide a feed additive and a probiotic agent, which contain said microorganism.

Still further object of the present invention is to provide a method for preparing a fermented food, which comprises fermenting food using said microorganism and a fermented food prepared by said method.

To achieve the above objects, in one aspect, the present invention provides the microorganism *Saccharomyces exiguous* SJP6728AF1 (KCCM-10675P).

In another aspect, the present invention provides a microbial agent for the fermentation of organic waste, which contains said microorganism.

In still further aspect, the present invention provides an agent for preventing or removing an offensive odor from organic waste, which contains said microorganism.

In yet further aspect, the present invention provides an insecticide, a microbicide and a preservative, which contains said microorganism.

In yet still another aspect, the present invention provides a feed additive or a probiotic agent, which contains said microorganism.

In still further aspect, the present invention provides a method for preparing a fermented food, the method comprises fermenting food using said microorganism, as well as a fermented food prepared through said method.

Other features and embodiments of the present invention will be more fully understood from following detailed description and the appended claims.

### BRIEF DESCRIPTION OF DRAWINGS

FIG 1 shows the insecticidal effect of the inventive microorganisms against fly larvae. In FIG 1, A illustrates photographs showing the insecticidal effects of the inventive microorganisms against six kinds of fly larvae, and B is a photograph enabling observation of the state of a fish left to stand for 3 days in an insecticidal test against said fly larvae.
FIG 2 shows the antibacterial and antifungal activities of the microorganisms according to the present invention.
FIG. 3 illustrates photographs of manures from livestock fed with feed containing the microorganisms according to the present invention. In FIG 4, A is a photograph of manure from livestock fed with feed containing an SJP6728AF1 culture broth, B is a photograph of manure from livestock fed with feed containing an SJP6729AF2 culture broth.
FIG. 4 shows photographs of garlic fermented using the microorganisms according to the present invention.
FIG. 5 shows photographs of ginseng fermented using the microorganisms according to the present invention.

### DETAILED DESCRIPTION OF THE INVENTION, AND PREFERRED EMBODIMENTS

In the present invention, microorganisms having the efficiency for removing the offensive odor of organic waste were first isolated in the following manner. Toxic substances were extracted from toxic plants, including *Aconiti ciliare, Aconitum carmichaeli, Quisqualis indica, Aconitum koreanum, Melia azedarah var. japonica, Styrax japonica,* etc. and spread on soil to induce the mutation of microorganisms present in the soil. The soil was applied to organic waste and, as a result, it was observed that the soil had odor removal efficiency. Then, 24 kinds of microorganism were isolated from the soil.

Among the above-isolated microorganisms, the microorganism SJP6728AF1 having the effects of removing the offensive odor of organic waste, killing fungi and insects and preventing decomposition was identified, and deposited in the Korean Culture Center of Microorganisms (KCCM), which is an international depository authority under the Budapest treaty.

Said SJP microorganism was excellent with respect to the ability to ferment organic materials, decomposition-preventing ability, odor-preventing ability and insecticidal/fungicidal abilities. Particularly, the microbial strain SJP6728AF1 identified as yeast was easily cultured in both anaerobic and aerobic environments and showed excellent abilities, except for fungicidal ability, compared to the abilities of the microbial strains (SJP6722A5, SJP6731B1, SJP6735B6, SJP6844AF5, SJP6732B2, SJP6719B3, SJP6734B4. SJP6841L2, SJP6720L3 and SJP6742L5) identified as the genus. *Bacillus.* Said effects varied depending on the compositions of culture media.

When said microorganism was continuously used in a specific system for 3 months or longer, the odor-preventing and harmful insect-controlling effects thereof were slowly decreased, but when said microorganism was used in other systems placed in other locations, it again showed the effects shown in the previous system. This indicates that the odor-preventing and harmful insect-controlling activities of said microorganism were relatively decreased due to microorganisms (putrefactive bacteria) having developed tolerance to specific microorganisms.

Also, when said microorganism was added to feed, digestion was promoted, and when the microorganism was used in the preparation of fermented foods, fermented foods having better efficiency than that of prior fermentation methods could be prepared.

The SJP yeasts according to the present invention, SJP6728AF1, belonging to the genus *Saccharomyces,* can be used in fermented foods. This is because yeasts, the use of which is accepted as being in compliance with the Korean food additive code provided by the Korean Food and Drug Administration, are limited to the genus *Saccharomyces.* The characteristics of the novel strain, SJP6728AF1, were compared with those of prior S. *exiguous* and, as a result, when S. *exiguous* was inoculated into food waste, an alcoholic odor was not generated even after 24 hours, but when food waste was treated with SJP6728AF1, an alcoholic odor could be sensed after 90 minutes.

### Examples

Hereinafter, the present invention will be described in further detail with reference to specific examples. However, those skilled in the art will appreciate that these examples are not intended to limit the scope of the present invention, and various changes and modifications are possible within the sprit and scope of the present invention.

### Example 1: Isolation and identification of novel microorganisms and insecticidal, microbicidal and odor-preventing effects thereof

### 1-1: Isolation of microorganisms

In order to screen microorganisms having strong tolerance to toxicity and strong survival ability and induce the mutation thereof, 300 g of toxic plants, including *Aconiti ciliare, Aconitum carmichaeli, Quisqualis indica, Aconitum koreanum, Melia azedarah var japonica,* and *Styrax japonica,* were diluted at the same amount, added to 3 liters of water, and subjected to hot-water extraction at 70-80°C for 2-3 hours, thus extracting a toxic substance.

Said extract and salt were periodically spread onto various soils, including barren soil, rich soil, leaf mold and vermicompost, for about 6 months, and then the soils treated with the extract were collected, and applied to food waste compost fermentation facility located at Shiheung-shi, Kyunggi-do, Korea. As a result, 12 kinds of offensive odors, including ammonia and sulfide hydrogen, were measured to be 0.00 ppm (Table 1).

**Table 1: Odors in food waste treatment plant**

| Test item | Standard (ppm) | Result (ppm) |
|---|---|---|
| Ammonia | ≤ 2 | 0.0 |
| Methylmercaptane | ≤ 0.004 | 0.000 |
| Hydrogen sulfide | ≤ 0.06 | 0.00 |
| Dimethylsulfide | ≤ 0.05 | 0.00 |
| Dimethyldisulfide | ≤ 0.03 | 0.000 |
| Trimethylamine | ≤ 0.02 | 0.000 |
| Acetaldehyde | ≤ 0.1 | 0.00 |
| Styrene | ≤ 0.8 | 0.03 |
| Propionaldehyde | ≤ 0.1 | 0.00 |
| Butyraldehyde | ≤ 0.1 | 0.000 |
| n-Valeraldehyde | ≤ 0.02 | 0.000 |
| i-Valeraldehyde | ≤ 0.006 | 0.000 |
| Bad smell | ≤ 20 | 8 times |

Also, in the food waste compost fermentation facility, composts were produced in an amount of 12-13 tons/day and the water content thereof was 65-70 %, but when the soils treated with the extract were applied to the plant, the production of composts was decreased to 5-6 tons/day, the water content thereof was 45-48%, and the volume thereof was also reduced to 1/3.

Moreover, when the extract-treated soils were applied to a container for separating and storing unrecyclable materials (vinyl bags, pig heads, fish, etc.) in the food waste compost fermentation facility, fly larvae were not generated.

In order to confirm microorganisms causing said effects, composts fermented with said extract-treated soils for 10 days and composts fermented with the soils for 30 days were collected, and analyzed at the Korean National Institute of Agricultural Science and Technology. As a result, 24 kinds of microorganisms, which were not present in prior composts untreated with the extract-treated soils, were detected and each of the microorganisms was isolated.

### 1-2: Insecticidal effect against fly larvae, food waste-fermentation effect and odor-preventing effect

Among said 24 kinds of microorganisms, 10 kinds of microorganisms were first randomly selected, and the insecticidal effect thereof against fly larvae and the odor-preventing effect thereof were then measured.

In the same manner as conventional methods of culturing yeasts, lactic acid bacteria or the genus *Bacillus* microorganisms, animals and plants containing nutrients, such as carbon sources, nitrogen sources, vitamins and minerals, were steamed at 121°C and extracted to prepare a culture medium, and each of said 10 kinds of microorganisms was inoculated into the resulting culture medium and cultured at 30-45 °C for 24-62 hours. Solid microbial agents were prepared by inoculating each of said 10 kinds of microorganisms into organic solids, such as sterilized sawdust, rice bran, wheat bran, rice powder and corn powder and fermenting the inoculated organic solids.

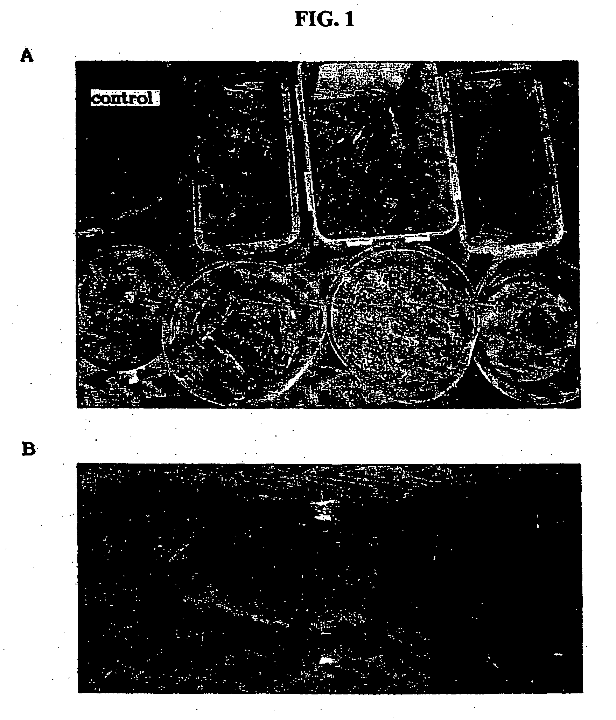

In order to measure the insecticidal effect of said microorganisms against fly larvae, 5 liters of rotten fish, chicken and pork were left to stand at room temperature for 4 days to generate fly larvae. Then, the fly larvae were placed in each of the containers at a density of a minimum of about 500-1,000 larvae, and 10 ml of each of the 10 kinds of microorganism culture broth was added to 100 ml of water and applied to each of the containers. Then, the time and state, at which the fly larvae were killed, were examined. As a result, when the food wastes were treated with SJP6728AF1 the fly larvae were killed (Table, 2 and FIG. 1).

**Table 2: Insecticidal effect against fly larvae**

| Microorganisms | Insecticidal rate(%) | Time (h) | Degrees of bad smell and the state of fly larvae |
|---|---|---|---|
| SJP6728AF1 | 100 | 4 | 3 days after death, fly larvae were decomposed to become clean water. After 5 days, the state of fish was changed weakly and it had a weak odor. |

In order to measure an effect of said microorganism on the fermentation of food waste, 20 ml of microorganism culture broth was spread onto 20 liters of food waste having a water content of about 65%, followed by agitation. Then, the food waste was warmed to maintain a temperature of 40-50°C From 3 days after the microbial treatment, the degree of odor generation, was measured using a sensory test and an odor meter.

As a result, when the food waste was treated with the SJP6728AF1 microbial strain, the freshness thereof was not changed and it was odorless (Table 3). This indicates that SJP6728AF1 has an excellent ability to prevent decomposition.

**Table 3: Results of food waste fermentation**

| Microorganisms | Deodorization effect (%) | Period (day) | Sensory test result |
|---|---|---|---|
| SJP6728AF1 | 100 | 3 | odorless |

Also, in order to measure the odor-preventing effect of said microorganisms, 5 ml of microorganism culture broth was applied to wastewater (BOD 100,000 ppm) generated in food waste, and a change in the offensive odor of the wastewater was measured using an odor meter.

As a result, when the wastewater was treated with SJP6728AF1, more than 90% of the offensive odor thereof was removed 90 minutes after the microbial treatment, and the offensive odor thereof was not generated even 6 days after the microbial treatment (Table 4).

**Table 4: Odor of food wastewater**

| Microorganisms | Deodorization effect (%) | Time (min) | Effect | Alcohol conc. (%) |
|---|---|---|---|---|
| SJP6728AF1 | 90 | 90 | After 90 min, bad odor disappeared and alcohol smell was generated | 8 |

Before and after collecting food waste into food waste collection containers, 20-50 ml of microorganism culture broth (SJP6728AF1) was spread into the food waste, the offensive odor of the wastewater was not generated not only in the collection containers, but also in collection vehicles and pretreatment systems, and the offensive odor thereof and fly larvae were not generated even when the food waste was not collected for 3-5 days (a maximum of 10 days).

### 1-3: Measurement of antibacterial and antifungal activities

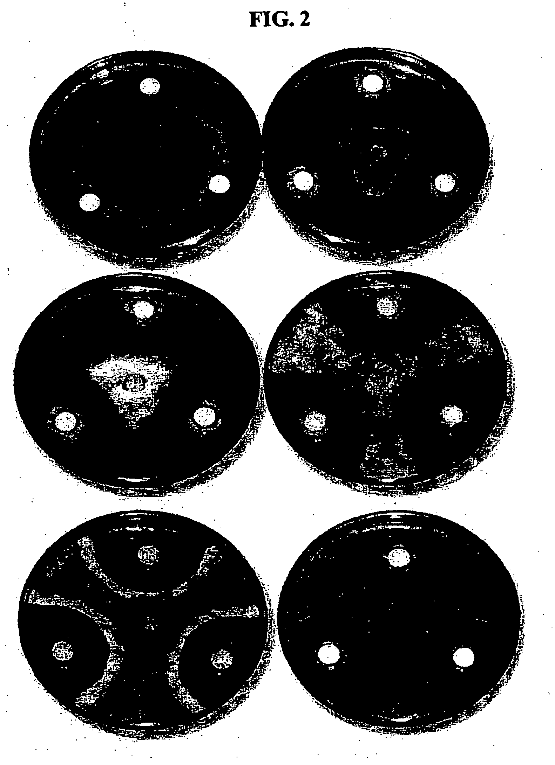

The antibacterial and antifungal activities of said microorganism (SJP6728AF1) against plant pathogenic bacteria were measured at the Plant Pathological Department, the Biological Division, the Korean National Institute of Agricultural Science and Technology. Bacteria and fungi were inoculated into SDA (Sabouraud dextrose agarblock) and cultured for 48 hours. Then, a block immersed in said microbial culture broth for 5 minutes was inoculated into the media having the bacteria and fungi cultured therein, and were cultured at 15°C in a dark condition for 24 hours, and the diameter of colonies formed by the bacteria was measured in units of mm. As a result, the antibacterial and antifungal activities of SJP6728AF1 microorganisms were shown to be high (Table 5 and FIG. 2).

**Table 5: Antibacterial and antifungal activities of SJP microorganisms**

| Bacteria | Activities |
|---|---|
| | AF1 |
| *Agrobacterium vitis* | - |
| *Clavibacter michiganensis subsp. michiganensis.* | + |
| *Pectobacterium carotovorum* subsp. *carotovorum* | - |
| *Xanthomonas campestris* pv. *campestris* | - |

| Fungi | |
|---|---|
| *Colletotrichum gloeosporides* | - |
| *Fusarium oxysporum* | - |
| *Phytophthora capsici* | - |
| *Rhizoctonia solani* | + |
| *Sclerotinia sclerotiorum* | + |
| Activity grade: - non-activity + weak ++ moderate +++ excellent | |

### 1-4: Identification of microorganism

SJP6728AF1 was identified at the Korean Culture Center of Microorganisms (KCCM) and, as a result, the 18S rDNA of SJP6728AF1 (SEQ ID NO: 1) showed a homology of 97% to *Saccharomyces exiguus*. Said microbial strain was deposited in the Korean Culture Center of Microorganisms (KCCM) (Table 6).

**Table 6: Name and deposit number of SJP microorganism**

| Name | Deposit number |
|---|---|
| SJP6728AF1 | *Saccharomyces exiguous KCCM-10675P* |

Microbial strains most similar to the inventive microorganism were purchased or distributed from the Korean Agricultural Culture Collection (KACC), the Korean Culture Collection of Microorganisms (KCCM), and the Korean Collection for Type Cultures (KCTC). Whether the distributed microbial strains have an insecticidal effect against fly larvae, an odor-preventing effect and an organic waste-fermentation effect was examined according to the above-described methods (Table 7 and Table 8).

**Table 7: Insecticidal effect against fly larvae**

| Similar microorganisms | Insecticidal rate (%) | Time of death (h) |
|---|---|---|
| *Candida zeylanoides, Candida humilis* | 0 | 12 |
| *Lactobacillus casei, Lactobacillus brevis, Camobacterium maltaromaticum, Brevibacillus borsterensis* | 10∼20 | 9 |
| *Paenibacillus* sp., *Paenibacillus lactis, Lactobacillus ctreum* | 20∼30 | 6 |

**Table 8: Fermentation effect of food waste**

| Similar microorganisms | Fermentation effect (%) | Fermentation period (day) |
|---|---|---|
| *Candida zeylanoides, Candida humilis, Kazachstania aerobia* | 50 | 3 |
| *Camobacterium maltaromaticum, Brevibacillus borsterensis* | 30 | 3 |
| *Lactobacillus casei, Lactobactilus brevis, Paenibacillus* sp., *Paenibacillus lactis, Lactobacillus ctreum* | 10 | 3 |

The results of examination of the insecticidal effect against fly larvae showed that the case of treatment with *Paenibacillus* sp. showed a death rate of 30%, but vigorous fly larvae regeneration occurred again only 10 hours after the microbial treatment. Also, the odor-preventing effects of the distributed microorganisms were significantly lower than those of the case treated with microbial strain SJP6728AF1 according to the present invention.

### Example 2: Analysis of composts fermented with SJP microorganisms

Composts fermented using the microorganism according to the present invention for 10 days and 30 days were not thermally treated. On the other hand, composts fermented in these conditions were thermally treated at 70°C for 10 minutes. Then, the density of the microorganism in the composts was measured using a streak plate method (Table 14).

As a result, in the fermented composts treated with the inventive microorganism, microorganisms were detected in an amount about 12-56 times larger than those in the control group, and the density of fungi was relatively low. However, in the composts fermented using the inventive microorganisms for 10 days, the density of yeasts was high compared to the control group.

**Table 9: Density of microorganisms in compost samples (unit: x10⁶ CFU/g)**

| Samples | Bacteria | | Fungi | Yeasts |
|---|---|---|---|---|
| | Non-heat treatment | Heat treatment (70°C, 10 min) | | |
| The inventive microorganism treated compost (10day fermentation) | 56.0 | 1.0 | 0.07 | 0.32 |
| The inventive microorganism treated compost (30day fermentation) | 12.3 | 2.4 | 0 | 0 |
| General compost | 1.1 | 0.1 | 0.73 | 0 |

Also, it was found that microorganisms detected in the composts treated with the inventive microorganism consisted mainly of the genus *Bacillus* and yeasts, microorganisms having an odor reduction effect and an insecticidal effect against fly larvae were detected in the composts treated with the inventive microorganism, and microorganisms having an antibacterial activity against plant pathogenic bacteria, and microorganisms promoting the growth of plants, were detected in the composts treated with the inventive microorganism. This indicates that the microorganisms having an effect on odor reduction can be used in food waste and livestock manure, and the microorganisms having an antibacterial activity, a plant growth-promoting effect and an insecticidal effect against fly larvae can be used as agents for controlling disease and insect pests.

Also, the microorganism according to the present invention showed effects varying depending on medium compositions. In other words, when microorganisms according to the present invention were inoculated and cultured in a medium made of only rice bran, and the insecticidal effect thereof against fly larvae was then measured, SJP6728AF1 were was most effective.

### Example 3: Odor-preventing effect

The odor-preventing effects of microorganisms (SJP6728AF1) according to the present invention were analyzed at the Department of Earth & Environmental Sciences, Sejong University. After making fish and meats rotten, said microorganisms were inoculated into the rotten fish and meats. Then, a change in the offensive odor of the fish and meats was measured using an odor meter for 30 days after the microbial treatment.

As a result, the offensive odor of the fish and meats was several thousand-fold reduced 1 hr ∼ 1 day after the microbial treatment. The offensive odor was reduced with the passage of time and reduced to 1/7 after 7 days. Particularly, hydrogen sulfide decreased by 99.99% so that there was no trace of the offensive odor, and the offensive odor was generated again after 30 days. However, it could be seen that the inventive microorganisms are effective in preventing odors, considering that most organic wastes are disposed of within 2-3 days. Also, because the inventive microorganisms show an odor-preventing effect and a decomposition preventing effect for 30 days, they are useful as preservatives for maintaining the freshness of fish, vegetable and the like.

### Example 4: Tests on cattle, pig and chicken feeds

Solid organic materials, such as rice bran, wheat bran and soybean, were mixed with water to a water content of 65-70%, and agitated such that water was uniformly absorbed into the organic materials. The agitated materials were sterilized with steam, and then inoculated with said microorganism according to the invention. The resulting materials were cultured for 30-40 hours, dried and milled, thus preparing feed additives.

In another method, according to the present invention was cultured in a liquid broth, and then mixed with solid organic materials such as rice bran, wheat bran and soybean, thus preparing livestock feed additives. In still another method, according to the present invention was cultured in a liquid broth and dried, and then the dried microorganisms were mixed with solid organic materials, such as rice bran, wheat bran and soybean, thus preparing feed additives or feeds.

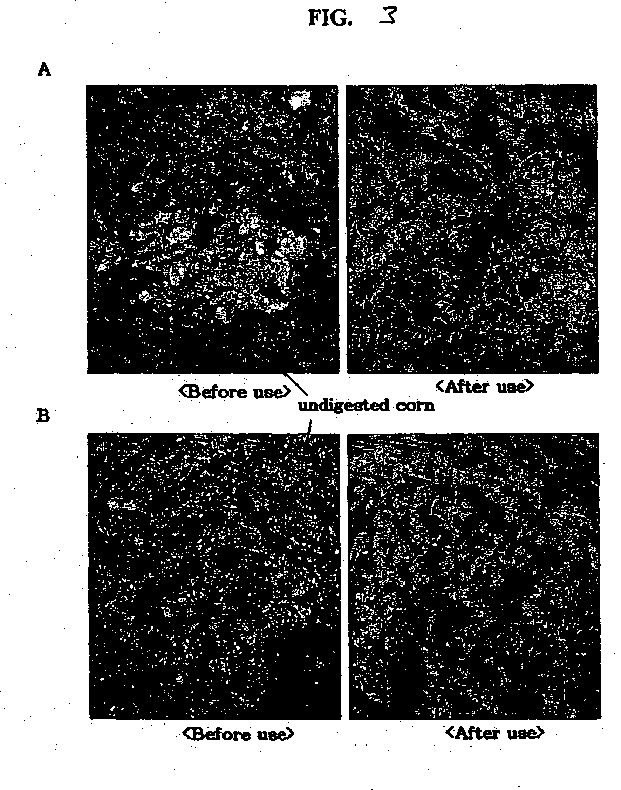

In the cattle manure of control group, which was not fed with the feed additives or water additives prepared as described above, corn used as cattle feed was evacuated from the bowels without digestion, but in the manure of cattle fed with the feed additives or water additives, corn used as feed was not seen (FIG. 3). This indicates that the SJP microorganisms according to the present invention have the effect of promoting digestion.

Also, plants such as maize and *Scutellaria baicalensis,* which are rich in yellow pigments (xanthophyll, carotene, etc.), tend to show a deeper yellow color when they are fermented. Thus, when the SJP microorganism culture broth according to the present invention were added to chicken feed, the skin of the resulting chicken, the shell of the eggs, and the yellow eggs, showed a deeper yellow color.

### Example 5: Substitution for growth-promoting antibiotics in pig feed

The verification of the antibiotic-substitution, growth-promoting effect, feed-saving effect and odor- and fly-preventing effects of the inventive microorganisms was conducted on pigs. 40% rice bran, 30% wheat bran and 30% mixture of red pepper seeds, *Scutellaria baicalensis,* ginger, cinnamon and licorice were powdered, sterilized, and cultured with SJP6728AF1, thus preparing feed additives. Pigs were fed with the feed additives to confirm whether the microorganisms can substitute for antibiotics. Finishing pigs (50.5 kg) were allotted to three treatment groups (four replications per treatment), and in order to reduce a deviation in body weight and an error caused by the division between male and female pigs, total test pigs were divided into four groups (two female groups and two castrated male groups) according to body weight and sex. In a control group, antibiotics (55 ppm neomycin + 110 ppm terramycin) were added. Also, an antibiotic-free control group was used, and the culture broth of the inventive microorganisms was added to water at a concentration of 2.5%, and then the pigs were fed with water to confirm the effect of substituting for antibiotics (Table 10).

**Table 10: Substitution test of antibiotics with SJP microorganisms**

| | Weight gain (g) | Feed intake (g) | Feed efficiency^{a} | Remark |
|---|---|---|---|---|
| Antibiotic feed control | 840.9 | 1908.0 | 2.28 | 20 pigs (female and male) 4 repetition |
| Non-antibiotic feed control | 695.4 | 1830.0 | 2.61 | 20 pigs (female and male) 4 repetition |
| SJP microorganism treatment group Without antibiotics | 818.8 | 1839.1 | 2.25 | 20 pigs (female and male) 4 repetition |

| | | | | |
|---|---|---|---|---|
| ^{a}Feed efficiency^{a} = Feed intake / Weight gain | | | | |

As a result, the group fed with the feed additive inoculated with the inventive SJP microorganisms without treatment with antibiotics showed a daily body weight gain similar to that of the control group fed with the antibiotic-containing feed, and a feed efficiency of 2.25 similar to that of the control group fed with the antibiotic-containing feed. This suggests that the microorganisms according to the present invention are useful as antibiotic substitutes.

Also, the effects of the SJP microorganisms according to the present invention on the number of bacteria in manure and the generation of odor were analyzed. The manure of livestock was collected before it falls onto soil, and the total bacterial number, the number of *E. coli* and the number of lactic acid bacteria in the collected manure were measured using a streak plate method. The amount of noxious gases generated was measured by analyzing ammonia and hydrogen sulfide using an odor analyzer, and the data were subjected to the analysis of variance using ANOVA of the SAS package. The test of significance between the groups was performed using Duncan's new multiple range test (Steel and Torrie), the confidence level was 95% (Table 11).

As a result, in the case where SJP microorganism culture broth were administered to pigs, there was no change in the total number of bacteria in the bowels of the pigs, but the number of *E. coli* as harmful bacteria was greatly reduced. Also, the results of analysis of noxious gases showed that, when the feed additive was treated with the inventive microorganisms, the generation of ammonia and hydrogen sulfide among noxious gases in the pig manure was decreased.

**Table 11: Number of bacteria and amount of harmful gases generated in manure upon addition of SJP microorganisms**

| Treatment | T1 (Antibiotic feed) | T2 (Non-antibiotic feed) | T3 (SJP addition without antibiotics) | LSD(0.05) |
|---|---|---|---|---|
| Number of bacteria | | | | |
| Total bacteria | 22.3 x 10⁶ | 35.2 x 10⁶ | 33.1 x 10⁶ | NS |
| *E. coli* | 37.2 x 10⁴ | 32.1 x 10⁴ | 9.3 x 10⁴ | 22.2 |

| Harmful gas (ppm) | | | | |
|---|---|---|---|---|
| Ammonia sulfide | 1.4 | 1.8 | 0.3 | 0.8 |
| Hydrogen | 49.0 | 31.5 | 0.1 | 22.0 |

### Example 6: Substitution for growth-promoting antibiotics in chicken feed

The effect of feed additives containing the inventive microorganisms SJP6728AF1 on the production of chicken was measured. 270 chickens were allotted to 3 treatment groups (3 replications per treatment), so that the animals were divided into a control group administered with a dilution of antibiotics (0.05% virginiamycin and 0.03% anticoccidium agent), and groups treated with 0.5% and 1.0% feed additives fermented using the SJP microorganism culture broth SJP6728AF1 according to the present invention. The animal groups were bred for 5 weeks to analyze the production of chicken (Table 12).

Also, mortality and growth ratio were analyzed according to the number of chickens killed during the chicken-raising period of 5 weeks (Table 13), the enteric microorganisms of the chickens were analyzed (Table 14), and the offensive odor of the chicken manure was measured using an odor meter (Table 15).

**Table 12: Effect of SJP microorganism-containing feed additive on production of chicken.**

| | Antibiotic feed control | 0.5% feed additive with SJP | 1.0% feed additive with SJP |
|---|---|---|---|
| Initial weight (g/number) | 46.1 | 46.8 | 46.7 |
| Final weight (g/number) | 1,507 | 1,537 | 1,582 |
| Weight gain (g/number) | 1,461 | 1,490 | 1,535 |
| Feed intake (g/number) | 2,344 | 2,300 | 2,376 |
| Feed demand ratio^{a} | 1.61 | 1.55 | 1.55 |

| | | | |
|---|---|---|---|
| ^{a} Feed demand ratio= Feed intake / Weight gain | | | |

**Table 13: Mortality and growth ratio of chicken**

| | Antibiotic feed control | 0.5% feed additive with SJP | 1.0% feed additive with SJP |
|---|---|---|---|
| Mortality (%) | 1.1 | 1.2 | 0 |
| Growth ratio (%) | 98.9 | 98.3 | 100 |

**Table 14: Number of enteric microorganisms (5 weeks)**

| | Antibiotic feed control | 0.5% feed additive with SJP | 1.0% feed additive with SJP |
|---|---|---|---|
| | Log₁₀ cfu/g | | |
| Total number | 8.122 | 7.797 | 7.924 |
| *E. coli* | 7.350 | 7.022 | 6.579 |
| *Lactobacillus sp.* | 8.322 | 8.463 | 8.717 |

**Table 15: Odors of chicken manure**

| | Antibiotic feed control | 0.5% feed additive with SJP | 1.0% feed additive with SJP |
|---|---|---|---|
| Ammonia (ppm) | 2.1 | 0.14 | 0.18 |
| Hydrogen sulfide (ppm) | 78.5 | 0.23 | 0.16 |

As a result, the groups treated with the inventive SJP microorganisms resulted in high production rate, low mortality, decreased feed amount, and decreased odors, compared to those of the group treated with the antibiotics. Also, the test pig meat and chicken meat were boiled in pure water and tasted by 50 persons. As a result, the 50 persons all evaluated that the inventive meat were soft, had reduced characteristic odor and good taste, compared to prior meats. Accordingly, treatment with the SJP microorganisms according to the present invention enables eco-friendly livestock products to be produced without using antibiotics.

### Example 7: Feed additives fermented with Chinese herbs

The SJP microorganisms according to the present invention survived upon application of natural insecticides made of toxic plants, and thus whether the inventive microorganisms can solve a problem of toxicity present in ginkgo leaves and Chinese herbs was examined. First, 200 g of ginkgo leaves were subjected to hot water extraction in 1.5 liters of water, and the extract was inoculated with SJP microorganism culture broth according to the present invention. The resulting extract was placed in a heating cabinet maintained at 40°C together with a control group non-inoculated with the inventive microorganisms, and then fermented for 24 hours. Then, whether gas was generated in the treated group and the control group was analyzed. As a result, gas was not generated in the control group, whereas gas was generated in the group treated with the inventive SJP microorganisms.

In the extract treated with said microorganisms according to the invention, gas was not generated for 7 days, and thus the fermentation of the extract was considered to be terminated. In order to examine whether the inventive microorganisms counteracted the poisonous effects, a toxicity test on the extract was performed by a sensory test by sensing the extract with the tongue in the mouth. As a result, the extract fermented with the inventive SJP microorganisms gave soft sensation without rejection. However, the non-fermented extract resulted in gnawing sensation, vomiting symptoms, a biting taste, and offensive toxic odors. Thus, when gingko leaves or Chinese herbal materials are treated with the SJP microorganisms according to the present invention, the toxicity of the gingko leaves or Chinese herbal materials can be reduced.

It was thought that, if ginkgo leaves are fermented and used as feed or feed additives for pigs, poultry and cattle, the medicinal component of the ginkgo leaves can be accumulated. Thus, ginkgo leaves were fermented in the following manner. Ginkgo leaves were inoculated and fermented with SJP microorganisms, dried and milled, thus preparing fermented ginkgo leaf compositions. *Sophora flavescens,* red pepper seed, licorice, cinnamon and *Scutellaria baicalensis* were diluted at the same amount, and inoculated with microorganisms according to the present invention. Then, the plants were placed in a heating cabinet maintained at 40°C together with a control group non-inoculated with the inventive microorganisms, and were then fermented, for 24 hours, thus preparing fermented Chinese herbal compositions.

The culture broth of the SJP microorganisms according to the present invention, and the Chinese herbal feed additive containing the fermented Chinese herbal composition and the gingko composition fermented with the SJP microorganism culture broth, were diluted in feed at a concentration of 1%. Chicken was fed with the feed, and the average body weight upon initiation of feeding and the average body weight gain after 8 days were examined.

As a result, the control group treated with the feed containing only the inventive SJP microorganism culture broth showed a body weight gain of about 400 g, but the group treated with the Chinese herb feed additive fermented with the SJP microorganism culture broth showed a body weight gain of about 600 g. Also, odors were not sensed at a distance of 2 m from the manure of all the treated livestock groups, and fly larvae were not substantially generated (50∼100%) (Table 16).

Thus, it could be seen that, when the feed additive obtained by fermenting ginkgo leaves and Chinese herbal materials with the SJP microorganism culture broth was fed, the growth of chicken was promoted, compared to when only the SJP microorganism culture broth according to the present invention was added to feed.

**Table 16: Growth-promoting, odor-preventing and fly larva-preventing effects of SJP microorganisms and Chinese herb feed additives fermented with SJP microorganism culture broth**

| | SJP culture broth (%) | Chinese herb feed additive (%) | Initial weight (g) | Final weight (g) | Bad smell | Fly larvae |
|---|---|---|---|---|---|---|
| SJP6728AF1 | 2 | 0 | 1410 | 1802 | NO | NO |

### Example 8: Effect of preventing decomposition of organic material

In order to examine the decomposition-preventing effect on the organic material of the SJP microorganism according to the present invention, bean curd was immersed in water, inoculated with said SJP microorganism, and left to stand at a temperature of 25-30°C. In the control group, odors began to be generated after 24 hours, but in the group treated with the SJP microorganism, odors were not sensed up to 3 days. After 5 days, some odors were generated in the yeast-treated group, and after 7 days, severe odors were generated.

The bean curd was taken out of water, and the odor and tissue of the bean curd were examined. As a result, it could be seen that odors were generated on the surface of the bean curd, but the inner part of the bean curd was maintained intact, and the tissue and firmness of the bean curd were the same as the first stage.

Also, a mackerel was inoculated with the SJP microorganism according to the present invention and left to stand at room temperature, and the odors thereof were examined After 1 day, the control group generated offensive odors. After 7 days, in the control group, fly larvae were generated. 7 days after the microbial treatment, the group treated with SJP6728AF1 started to generate odors. However, the groups treated with the yeast SJP microorganism according to the invention did not generate maggots even after 15 days. In the salted control group, some odors could be sensed after 15 days.

Soybeans were immersed in water for 2 hours, and then applied with the SJP microorganism culture broth according to the present invention. The treated group and the control group were left to stand at a temperature of 20-25°C. As a result, the control group began to generate mold and odor after 5 days. However, the group treated with the yeast SJP microorganism (SJP6728AF1) maintained the original state for 42 days, and slowly turned brown and became black after 60 days.

Also, soybeans were immersed in water for 10 minutes so that the soybeans adsorb water. Then, the feed additive powder prepared according to the method of Example 6 using the yeast SJP microorganism of the present invention was added to the soybeans at a ratio of 1:10 and left to stand. As a result, the soybeans maintained the original state even after 3 months. This suggests that the SJP microorganism according to the present invention can be used as a preservative for crops, fruits, vegetables, fish and shellfish.

### Example 9: Bean sprout cultivation test

The SJP microorganism culture broth according to the present invention was diluted in water, which was then watered to bean sprout, or the SJP microorganism culture broth was applied to bean sprouts 2-3 times a day when it is not the time for watering. As a result, in the group treated with the SJP microorganism culture broth, the growth of the bean sprouts was promoted without decomposition.

### Example 10: Fermentation of soybean, crops and beef bone broth and preparation of cheese

Soybeans were steamed, inoculated with SJP6728AF1 according to the present invention, and fermented for 30 hours. Then, the fermented soybeans were dried and powdered, thus preparing an enzyme food supplement. Then, 50 persons over 50 years old were selected and allowed to eat the fermented soybean enzyme food.

As a result, the soybeans fermented with SJP6728AF resulted in a nutty taste and fragrance, like roasted soybean flour, and most of the test subjects answered that they did not sense offensive odors upon a fart and excretion for 30 days of ingestion of the fermented soybeans, and the fermented soybeans had excellent digestion promoting effect and recovery effect from fatigue (Table 17).

**Table 17: Effects of ingestion of soybean food fermented with the SJP microorganism**

| Promotion of digestion | Bad smell of gas | Bad smell of excrement | Constipation solution | Recovery from fatigue | Energizing effect | Diet |
|---|---|---|---|---|---|---|
| 100% | 80% | 70% | 90% | 90% | 60% | 30% |

Also, cereals, such as unpolished rice, barley, wheat, bean and sesame, were mixed with each other at the same ratio, steamed, and then inoculated with SJP6728AF1. Then, the mixture was fermented at 35-40°C for 2 days, kneaded and made into enzyme pills. When the pills were administered into persons, they had effects on digestion promotion, removing fecal stasis and odor prevention.

Beef bone broth obtained by degrading beef bones such as beef feet in hot water was inoculated with the SJP microorganism culture broth according to the present invention and fermented for 2 days. As a result, the characteristic odor of beef bone broth disappeared, and the color thereof was clear.

Sterilized milk was inoculated with the SJP microorganism according to the present invention, and then fermented for 12 hours. When purely white cheese was coagulated, it was dewatered and tasted. As a result, the cheese had a sour taste and nutty taste, which are characteristic of microbial fermentation, and it had effects on digestion promotion and odor prevention upon excretion. Also, the cheese was placed in a heating cabinet at 40°C, and after 2 days, yeasts were grown, but the taste of the cheese was not changed. In a conventional cheese preparation method, there are problems in that a large amount of offensive odors occurs and a fermentation process must be conducted for a long time; however, the cheese fermented using the SJP microorganism according to the present invention had no offensive odor, and did not give a greasy taste even when it was eaten after coagulation. Also, the inventive cheese had a nutty taste.

### Example 11: Preparation of bean curd

A bean extract obtained by steaming bean juice and removing bean curd dregs was adjusted to 40.5°C and then inoculated with SJP6728AF1 culture broth. Then, the bean extract was fermented at 40°C for 12 hours, and soft bean curd was prepared therefrom. According to a conventional bean curd dewatering process, the soft bean curd was placed into a bag and dewatered for 6 hours while being pressed under a weight of about 10 kg, thus preparing bean curd.

The bean curd thus prepared was tasted and, as a result, it had the same taste as that of conventional bean curd, but had a sour taste, indicating fermentation.

5 liters of the bean extract was added to 5 liters of raw milk, and the mixture was inoculated with the SJP microorganism according to the present invention and fermented for 24 hours, thus preparing a fermented semi-solid product made of the cheese/bean curd mixture.

Chocolate was added to the bean extract, the mixture was fermented and, as a result, chocolate bean curd was produced which had no sour taste. A pine leaf extract was added to raw milk, the mixture was fermented and, as a result, a cheese having pine leaf fragrance was produced. Also, salt and peach drink was added to the bean extract, the mixture was fermented and, as a result, the resulting product had peach fragrance and had no sour taste. Accordingly, mugwort and green tea can be used as food materials capable of harmonizing the taste and fragrance of said bean curd and cheese, and thus the inventive microorganism can be used in a significantly large range of applications.

The bean curd treated with the inventive SJP microorganism and conventional bean curd, were immersed in water and left to stand in a heating cabinet maintained at 40°C, and whether the bean curds were spoiled was examined. As a result, the conventional bean curd generated spoiled bean curd odors after 24 hours. However, the bean curd treated with the SJP microorganism according to the invention was not spoiled even after 5 days, and the surface thereof was covered with grown yeasts. Accordingly, it was expected that the decomposition prevention effect of the inventive SJP microorganism would last for at least 10 days. Also, when the bean curd or cheese treated with the SJP microorganism according to the invention was stored m a refrigerator, the offensive odors generated in the refrigerator disappeared.

### Example 12: Garlic fermentation

To measure the fermentation efficiency of the SJP microorganism according to the present invention, 5 kg of garlic was added to 30 liters of water, heated to 130°C and then cooled to 30°C. The resulting garlic solution was inoculated with the SJP6728AF1 culture broth, and then left to stand at room temperature for 30 days. As a result, the garlic was fermented such that the odor thereof was not sensed.

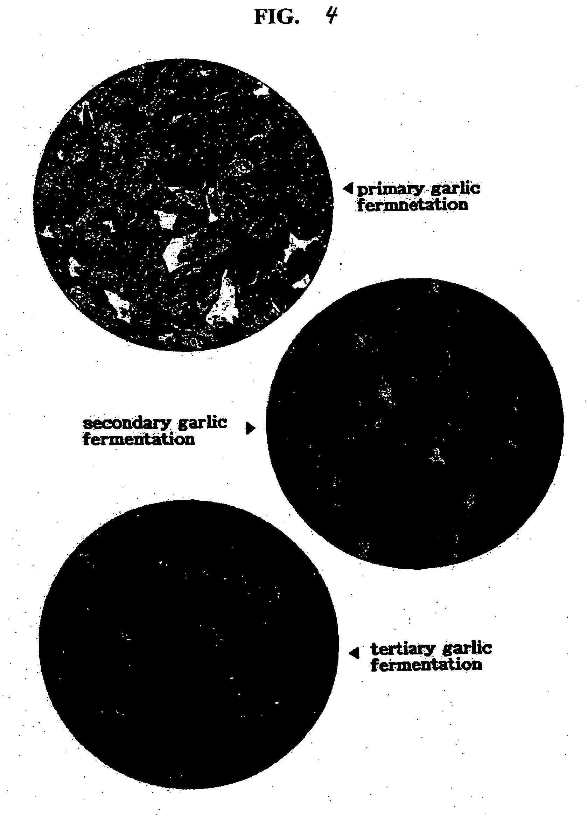

Garlic was steamed in a solid state and inoculated with the SJP6728AF1 culture broth. Then, the garlic was fermented at 35-40°C for 2 days and dried for 2 days, and the fermentation and drying process was repeated three times. As a result, the garlic was turned red in primary fermentation, and black in secondary fermentation and tertiary fermentation (FIG. 4). The black garlic showed a reduction in the characteristic odor of garlic or the hot taste of allicin, and thus it is expected that the fermented enzyme of the garlic can be easily administered and be usefully used in cancer therapy.

### Example 13: Ginseng fermentation

300 g of ginseng powder obtained by drying and milling 6-year-old fresh ginseng was inoculated with the SJP microorganism culture broth according to the present invention and fermented at 35-40 °C for 10 days. The fermented ginseng was steamed and the components of the fermented ginseng were measured with HPLC.

The crude saponin content of the control group was 5.12 w/w%, and the Rb1 content thereof was 0.037 w/w%, whereas the crude saponin content of the fermented ginseng inoculated with the inventive SJP microorganism culture broth was 5 w/w%, and the Rb1 content thereof was 0.538 w/w% (Table 18). Table 19 shows the measurement results of the water content of fresh ginseng before and after treatment with the inventive SJP microorganism culture broth, and Table 20 shows the results of HPLC analysis of fresh ginseng.

**Table 18: Components of ginseng fermented by the SJP microorganism according to the invention**

| Test item | Content (w/w%) |
|---|---|
| Crude saponin | 5.000 |
| Ginsenoside Rh2 | - |
| Rh1 | 0.538 |
| Rg2 | 1.111 |
| Rg3 | 1.769 |
| Rg1 | 11.212 |
| Rf | 2.338 |
| Re | 2.044 |
| Rd | - |
| Rc + Rb2 | 7.935 |
| Rb1 | 14.743 |

**Table 19: Water content of fresh ginseng**

| | Before treatment with SJP culture broth (g) | After treatment with SJP culture broth (g) | Water content (%) |
|---|---|---|---|
| Head | 0.635 | 0.165 | 74.0 |
| Major root | 9.592 | 2.999 | 68.7 |
| Rootlet | 4.046 | 1.268 | 68.7 |

**Table 20: Results of HPLC analysis of fresh ginseng**

| | Ethanol extract (g) | Crude saponin (%) | Total saponin (%) | Rb1/Rg1 | PD/PT | Ginseniside (w/w%) | | | | | | |
|---|---|---|---|---|---|---|---|---|---|---|---|---|
| | | | | | | Rb1 | Rb2 | Rc | Rd | Re | Rf | Rg |
| Total | 5.071 | 1.200 | 0.210 | 1.290 | 1.722 | 0.037 | 0.043 | 0.034 | 0.019 | 0.044 | 0.005 | 0.029 |
| Head | 0.149 | 1.888 | 0.218 | 1.309 | 1.526 | 0.047 | 0.043 | 0.030 | 0.012 | 0.045 | 0.005 | 0.036 |
| Major root | 3.423 | 0.737 | 0.055 | 0.929 | 0.964 | 0.010 | 0.007 | 0.007 | 0.002 | 0.012 | 0.004 | 0.011 |
| Rootlet | 1.192 | 1.477 | 0.296 | 2.737 | 2.325 | 0.059 | 0.068 | 0.050 | 0.030 | 0.063 | 0.004 | 0.022 |

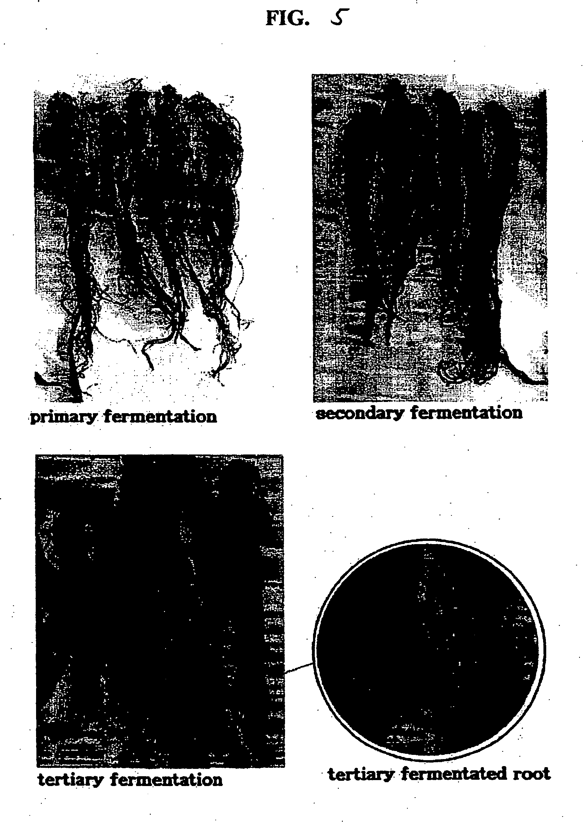

This suggests that, when fresh ginseng inoculated with the SJP microorganism according to the present invention is fermented, red ginseng extracts can be prepared in an amount more than two times larger than that of a conventional method for preparing red ginseng extracts. Particularly, even the fine root of the fresh ginseng fermented with the SJP6728AF1 culture broth could be prepared into red ginseng as shown in FIG. 5.

### Example 14: Preparation of rice bran beverage and fermentation of animal extract and fruit juice

An extract obtained by extracting rice bran at 121°C was inoculated with the SJP6728AF1 culture broth and fermented for 48 hours. Then, the extract was sterilized and adjusted to a sugar content of 11.5-12.5% by adding sugar thereto, so that it had a sweet and sour taste and the characteristic fragrance of the SJP microorganism according to the invention, and thus another flavor did not need to be added thereto. However, when the extract was fermented after adding fragrant grass such as pine leaves, peppermints, herbs, lemons and green tea thereto, yeast beverages having various fragrances could be prepared, and when the yeasts were isolated and purified by centrifugation, enzyme beverages having an unchanged taste could be prepared.

Also, 8 kg of fresh-water eels, soft-shelled turtles, crucians, deer and snake fish were mixed with 500 g of licorice root, and the mixture was extracted, inoculated with SJP6728AF1, fermented for 2 days, and then sterilized at high temperature, thus preparing a food product. When the food was ingested, it provided an energy restoration effect.

Commercially available beverages made with oranges, pears, peaches, apples, carrots, tomatoes, pomegranates and grapes were purchased in the market and the sugar content and pH thereof were measured. The measurement results showed that the sugar content was 11.5-12.5%, and the pH was 3.3-3.8. The commercial beverages were inoculated with SJP6728AF1 and placed in a fermenter maintained at 35-40°C. After 24 hours of fermentation, the pH and sugar content of the fermented beverages were measured and, as a result, the pH was 3.3-3.8, which was the same as the pH of the non-fermented beverages, but the sugar content was 10-11.5%, which was about 1-1.5% lower than the sugar content of the non-fermented beverages. The fermented beverages were further fermented for 24 hours and the pH and sugar content thereof were measured and, as a result, the pH was not changed, but the sugar content was further decreased by about 2-3%. Also, the further fermented beverages smelled of alcohol and had a sour taste stronger than that of the 24 hours fermented beverages. The fermented beverages were sterilized by heating, were adjusted to a sugar content of 11.5-12.5% by adding sugar thereto, and were tasted. As a result, fruit beverages were prepared, in which fruit fragrance was stronger than that of the non-fermented beverages and which had a sweet taste together with a sour taste.

### Example 15: Fermentation of Chinese herbal materials

In order to examine whether the bitter taste of Chinese herbal materials is changed when the Chinese herbal materials are fermented using the inventive SJP microorganism 500 g of *Phellinus* /*iteus* was added to 15 liters of water and subjected to hot water extraction. The extract was inoculated with SJP6728AF1 and then placed in a fermenter maintained at 35-40°C. After 24 hours of fermentation, the extract was observed. As a result, a great amount of gas was generated, suggesting that the fermentation of the extract progressed. After 2 days of fermentation, the taste of the fermented extract was compared with the control group non-inoculated with the SJP microorganism and, as a result, a bitter taste was not sensed in the SJP microorganism-treated group, but was maintained in the control group.

Also, Chinese herbs having high skin moisturizing effect, such as *Cnidium officinale, Angelica gigas* Nakai, and *Liriope platyphylla,* and animal protein such as eels, were inoculated with the SJP6128AF1 culture broth and fermented. Then, cosmetic materials such as ceramic powder were added thereto, and the mixture was massaged onto face for one week. As a result, the facial skin became soft, the face became bright, and a wrinkle improvement effect was shown.

Chinese herbs having effects against skin diseases, for example, *Sophora flavescens, Torilis japonica* and *Scutellariae baicalensis,* were inoculated with the SJP6728AF1 culture broth and fermented. Then, whether the herbs can treat athlete's foot was examined, and, as a result, the athlete's foot was perfectly cured when the herbs were administered 2-3 times, and the athlete's foot did not recur for 5 months.

Chinese herbs such as *Scutellariae baicalensis* were inoculated with the SJP6728AF1 culture broth and fermented, thus preparing fermented solution. When the fermented broth was used as bathing water, it showed the effects of treating not only athlete's foot, but also skin diseases, including atopic diseases.

Also, Chinese herbs such as *Pueraria lobata* and *Pueraria flos* were inoculated with the SJP6728AF1 culture broth and fermented, thus preparing beverages. When the beverages were ingested, they had an effect on removing hangover. *Chrysanthemum indicum* was inoculated with the SJP6728AF1 culture broth and fermented, thus preparing beverages. When the *Chrysanthemum indicum* beverages were ingested, they had the effects of removing headache pain and stabilizing blood pressure. An extract obtained by adding 6 kg of *Pueraria lobata* to 25 liters of water and subjecting the solution to hot water extraction was inoculated with the SJP6728AF1 culture broth and fermented for 5 days, and the sugar content thereof was measured. As a result, the sugar content was 5%. The fermented extract was adjusted to a sugar content of 10% by adding sugar thereto, and when it was ingested, it was effective at removing hangover.

3-year-old *Platycodon grandiflorum* solid was steamed, inoculated with the SJP6728AF1 culture broth and fermented for 2 days, and, as a result, the toxicity of the *Platycodon grandiflorum* disappeared. The *Platycodon grandiflorum* was fermented three times and dried three times, and thus it turned black. *Platycodon grandiflorum* has a problem in that it is not easily digested, but the black *Platycodon grandiflorum* can solve the problem of digestive absorption.

### Example 16 : Method of fermenting liquid organic material absorbed into solid organic material

300 g of *Saururus chinesis* and *Houttuynia cordata* were added to 3 liters of water, shaken for 2 hours, and then dewatered, thus obtaining 2 liters of a liquid organic material of *Saururus chinesis.* Soybeans were immersed in the above-prepared liquid organic material for 4 hours and, as a result, about 95% of the liquid organic material was absorbed into the soybeans. The resulting material was steamed, inoculated with the SJP6728AF1 culture broth, and fermented at 30°C for 48 hours. The fermented material was dried and milled, thus preparing a fermented composition. The fermented composition had low molecular weight, because the polymer components of *Saururus chinesis* were degraded by the microorganisms. Thus, the fermented composition has advantages in that the digestion efficiency thereof is increased and the nutrients of the fermented soybeans together with the microorganisms can be ingested.

Also, 300 g of fresh ginseng was added into 3 liters of water and subjected to hot water extraction, thus obtaining 2 liters of a liquid organic material of fresh ginseng. Black beans and black sesame were immersed in ShiQuanDaBuTang (hot water extract of ginseng, root of *Atractylodes japonica,* White poria cocos(Schw.) Wlof., licorice root, dried root of *Rehmannia glutinosa* Liboschitz var. *purpurea* Makino, *Paeonia japonica, Cnidium officinale, Angelica gigas* Nakai, *Astragalus membranaceus,* bark of *Cinnamomum cassia* Blume, date, and ginger) and steamed to prepare a semi-solid organic material. The semi-solid organic material was inoculated with the SJP6728AF1 culture broth and fermented for 3 days, thus preparing a fermented composition. As a result, the fermented composition obtained by inoculating the ShiQuanDaBuTang with the SJP6728AF1 culture broth and fermenting the inoculated material could have maximized potency, compared to when the ShiQuanDaBuTang was ingested in the form of a conventional hot water extract.

### INDUSTRIAL APPLICABILITY

As described in detail above, the inventive novel microorganism having the efficiency of removing an odor from organic waste has the effects of preventing or removing the odor from organic waste and preventing the decomposition of organic waste, and thus improve an environment. Also, the inventive microorganism has an insecticidal effect against noxious insects and an antifungal effect against plant pathogenic fungi, can be used as feed additives and antibiotic substitutes, and also are useful for the preparation of fermented foods.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. *Saccharomyces exiguous* SJP6728AF1 (KCCM-10675P).

2. A microbial agent for the fermentation of organic waste, which contains *Saccharomyces exiguous* SJP6728AF1 (KCCM-10675P).

3. An agent for preventing or removing an offensive odor from organic waste, which contains *Saccharomyces exiguous* SJP6728AF1 (KCCM-10675P).

4. An insecticide, which contains *Saccharomyces exiguous* SJP6728AF1 (KCCM-10675P).

5. A microbicide, which contains *Saccharomyces exiguous* SJP6728AF1 (KCCM-10675P).

6. A preservative, which contains *Saccharomyces exiguous* SJP6728AF1 (KCCM-10675P).

7. A feed additive, which contains *Saccharomyces exiguous* SJP6728AF1 (KCCM-10675P).

8. A method for preparing a fermented food, the method comprises fermenting a food using *Saccharomyces exiguous* SJP6728AF1 (KCCM-10675P).

9. A fermented food prepared by the method of claim 8, which contains *Saccharomyces exiguous* SJP6728AF1 (KCCM-10675P).

10. A probiotic agent, which contains *Saccharomyces exiguous* SJP6728AF1 (KCCM-10675P).

## Patentansprüche

1. *Sacharomyces exiguous* SJP6728AF1 (KCCM-10675P).

2. Mikrobielles Mittel zur Fermentation von organischem Abfall, das *Sacharomyces exiguous* SJP6728AF1 (KCCM-10675P) enthält.

3. Mittel zur Verhinderung oder Entfernung eines widerwärtigen Geruchs von organischem Abfall, das *Sacharomyces exiguous* SJP6728AF1 (KCCM-10675P) enthält.

4. Insektizid, das *Sacharomyces exiguous* SJP6728AF1 (KCCM-10675P) enthält.

5. Mikrobizid, das *Sacharomyces exiguous* SJP6728AF1 (KCCM-10675P) enthält.

6. Konservierungsmittel, das *Sacharomyces exiguous* SJP6728AF1 (KCCM-10675P) enthält.

7. Nahrungsmittelzusatz, der *Sacharomyces exiguous* SJP6728AF1 (KCCM-10675P) enthält.

8. Verfahren zur Herstellung eines fermentierten Nahrungsmittels, das das Fermentieren eines Nahrungsmittels unter Verwendung von *Sacharomyces exiguous* SJP6728AF1 (KCCM-10675P) umfasst.

9. Durch das Verfahren nach Anspruch 8 hergestelltes fermentiertes Nahrungsmittel, das *Sacharomyces exiguous* SJP6728AF1 (KCCM-10675P) enthält.

10. Probiotisches Mittel, das *Sacharomyces exiguous* SJP6728AF1 (KCCM-10675P) enthält.

## Revendications

1. *Sacharomyces exiguous* SJP6728AF1 (KCCM-10675P).

2. Un agent microbien pour la fermentation des déchets organiques, l'agent microbien contient *Sacharomyces exiguous* SJP6728AF1 (KCCM-10675P).

3. Un agent pour prévenir ou éliminer une odeur contrariante des déchets organiques, l'agent contient *Sacharomyces exiguous* SJP6728AF1 (KCCM-10675P).

4. Un insecticide que contient *Sacharomyces exiguous* SJP6728AF1 (KCCM-10675P).

5. Un microbicide que contient *Sacharomyces exiguous* SJP6728AF1 (KCCM-10675P).

6. Un agent conservateur que contient *Sacharomyces exiguous* SJP6728AF1 (KCCM-10675P).

7. Un additif alimentaire que contient *Sacharomyces exiguous* SJP6728AF1 (KCCM-10675P).

8. Un procédé pour la production d'un aliment fermenté, le procédé comprend fermenter un aliment au travers du *Sacharomyces exiguous* SJP6728AF1 (KCCM-10675P).

9. Un aliment fermenté fabriqué par le procédé selon la revendication numéro 8 que contient *Sacharomyces exiguous* SJP6728AF1 (KCCM-10675P).

10. Un agent probiotique que contient *Sacharomyces exiguous* SJP6728AF1 (KCCM-10675P).


### SEQUENCE LISTING

<110> Park, Se Joon
<120> Microorganisms having bad smell removal activity of organic waste and use thereof
<130> P 0217 WOEP
<140> EP 06799344.4
   <141> 2006-10-19
<150> KR 20050099940
   <151> 2005-10-22
<150> KR 20050103923
   <151> 2005-11-01
<150> KR 20060093709
   <151> 2006-09-26
<150> KR 20060093724
   <151> 2006-09-26
<150> KR 20060093713
   <151> 2006-09-26
<150> KR 20060094706
   <151> 2006-09-28
<150> KR 20060094687
   <151> 2006-09-28
<150> KR 20060098303
   <151> 2006-10-10
<160> 17
<170> PatentIn version 3.1
<210> 1
   <211> 1699
   <212> DNA
   <213> Saccharomyces exiguus
<400> 1
   ggccaagcgt tagttagcat ttatacgtga aactgcgaat ggctcattaa atcagttatc      60
   gtttatttga tagttccttt actacatggt ataactgtgg taattctaga gctaatacat     120
   gcttaaaatc tcgacctctg gaagagatgt atttattaga taaaaaatca atgtcttcgg     180
   actctttgat gattcataat aacttttcga atcgcatggc cttgtgctgg cgatggttca     240
   ttcaaatttc tgccctatca actttcgatg gtaggatagt ggcctaccat ggtttcaacg     300
   ggtaacgggg aataagggtt cgattccgga gagggagcct gagaaacggc taccacatcc     360
   aaggaaggca gcaggcgcgc aaattaccca atcctaattc acggaggtag tgacaataaa     420
   taacgatacc gggcccattc gggtcttgca tttggaatga gtactatgta aataccttac     480
   tgagcaatac tccgacgcca agtctgttgc cagcagccgc gaaaattcca gctccaatag     540
   cgtatattaa agttgttgca gttaaaaagc tcgtagatga actttgagtc tgtttggccg    600
   gcccgatttt tctccgtact ggcatcccaa gcggaccttt ccttctggct aaccttgggt    660
   ccttgtggcc cctggcgaac caggattttt actttgaaaa aattagagtg ttcaaagcag    720
   gcgtattgct cgaatatatt agcatggaat aatagaatag gacgtttggt tctattttgt    780
   tggtttctag gaccatcgta atgattaata gggacggtcg ggggcatcag tattcaaatg    840
   tcagaggtga aattcttgga ttttttgaag actaactact gcgaaagcat ttgccaagga    900
   cgttttcatt aatcaagaac gaaagttagg ggatcgaaga tgatcagata ccgtcgtagt    960
   cttaaccata aactatgccg actagggatc gggtggtgtt tttttaatga cccactcggc   1020
   accttacgag aaatcaaagt ctttgggttc tggggggagt atggtcgcaa ggctgaaact   1080
   taaaggaatt gacggaaggg caccaccagg agtggagcct gcggcttaat ttgactcaac   1140
   acggggaaac tcaccaggtc cagacacaat aaggattgac agattgagag ctctttcttg   1200
   attttgtggg tggtggtgca tggccgttat tagttggtgg agtgatttgt ctgcttaatt   1260
   gcgataacga acgagacctt aacctactaa atagtggtgc tagcatttgc tggttgtcca   1320
   cttcttagag ggactatcgg tttcaagccg atggaagttt gaggcaataa caggtctgtg   1380
   atgcccttag acgttctggg ccgcacgcgc gctacactga cggagccagc gagtctaacc   1440
   ttggccgaga ggtcttggta atcttgtgaa actccgtcgt gctggggata gagcattgta   1500
   attattgctc ttcaacgagg aattcctagt aagcgcaagt catcagcttg cgttgattac   1560
   gtccctgccc tttgtacaca ccgcccgtcg ctagtaccga ttgaatggct tagtgaggcc   1620
   tcaggatctg cttagaggag ggggcaactc cacctcagag cggagaattt gacaaactgg   1680
   tcatagagaa taatattgg                                                 1699